# EUROPEAN PATENT SPECIFICATION

(11) **EP 1 994 484 B1**
(45) Date of publication and mention of the grant of the patent: **25.03.2015**
(21) Application number: 07716683.3
(22) Date of filing: 17.01.2007
(51) Int. Cl.: G06F 19/00, G06F 17/30

(54) **PLATFORM FOR INTEROPERABLE HEALTHCARE DATA EXCHANGE**
PLATTFORM FÜR DEN AUSTAUSCH KOMPATIBLER GESUNDHEITSDATEN
PLATE-FORME POUR L'ÉCHANGE INTERFONCTIONNEL DE DONNÉES DE SOINS DE SANTÉ

(30) Priority: 17.01.2006 US 759015 P; 28.02.2006 US 777147 P
(43) Date of publication of application: 26.11.2008
(73) Proprietor: Accenture Global Services Limited, Dublin 4 (IE)
(72) Inventor: MYERS, Scott, D., Reston, VA 20190 (US); CELI, John, S., Reston, VA 20190 (US); QUINN, John, F., Reston, VA 20190 (US); KELLY, Brian, J., Reston, VA 20190 (US); THOMPSON, Gale, E., Reston, VA 20190 (US); RUFFIN, Marshall, Reston, VA 20190 (US); WU, Garret, R., Reston, VA 20190 (US); ROMAN, Shawn, D., Reston, VA 20190 (US); WRIGHT, Amy, H., Reston, VA 20190 (US); TRONOSKI, William, J., Reston, VA 20190 (US); TRUSCOTT, Andrew, J., Reston, VA 20190 (US)
(74) Representative: Müller-Boré & Partner Patentanwälte PartG mbB
(86) International application number: PCT/US2007/001134
(87) International publication number: WO 2007/084502

(56) References cited:
- WO-A-2005/088506
- US-A1- 2004 083 217
- GRIEW A ET AL: "DEVELOPING THE DISTRIBUTED PATIENT RECORD USING CORBA TECHNOLOGIES" PROCEEDINGS. TOWARDS AN ELECTRONIC PATIENT RECORD. INTERNATIONAL SYMPOSIUM ON THE CREATION OF ELECTRONIC HEALTH RECORD SYSTEM AND GLOBAL CONFERENCE ON PATIENT RECORD, vol. 2, 13 May 1996 (1996-05-13), pages 255-263, XP000956355

## Description

### CROSS REFERENCE TO RELATED APPLICATIONS

This application claims priority under 35 U.S.C. § 119(e) from U.S. Provisional Patent Application Nos. 60/759,015 filed on January 17, 2006 and 60/777,147 filed on February 28, 2006.

### Field of the Invention

The present invention generally relates to an interoperable healthcare data exchange platform. More specifically, embodiments of the present invention provide a system and related method for seamlessly linking a plurality of disparate, remote health records sources to enable the real-time collection, processing and centralized storage of health records, along with enabling controlled access to the centralized storage.

### BACKGROUND OF THE INVENTION

In April 2004, in Executive Order 13335, President George W. Bush revealed his vision for the future of health care in the United States. The President's plan involves a health care system that puts the needs of the patient first, is more efficient, and is cost effective. This plan is based on the following tenets:
- Medical information follows consumers (patients) so that they are at the center of their own care
- Consumers (patients) choose physicians and hospitals based on clinical performance results made available to them
- Clinicians have consumers' (patients') complete medical history, computerized ordering systems, and electronic reminders
- Quality initiatives measure performance and support quality-based competition in the industry
- Public health and bioterrorism surveillance is seamlessly integrated into care
- Clinical research is accelerated and post-marketing surveillance will be expanded.

In order to achieve these and other desired benefits, the President has charged the Secretary of the Department of Health and Human Services (DHHS) with executing this vision and established the Office of the National Coordinator for Health Information Technology (ONCHIT) to develop and maintain a strategic plan to guide the nationwide implementation of interoperable Electronic Health Records (EHRs) in both the public and private health care sectors to facilitate health information exchange. A fully functional platform for health information exchange deployed on a regional or national basis could provide the framework for authorized, secure, timely, and accurate exchanges of health information among patients, clinicians and other providers and authorized entities.

A fully functional platform for health information exchange deployed on a regional or national basis could provide the framework for authorized, secure, timely, and accurate exchanges of health information among patients, clinicians and other providers and authorized entities. The solution proposed in the present invention, as described below, will allow the widespread exchange of health care information by maximizing interoperability among health care software applications, particularly EHRs. This approach could be used in support of a National Health Information Network (NHIN) or support Regional Health Information Organizations (RHIOs) and Regional Health Communities (RHCs).

At the same time, the global market for Health IT is large and expected to grow rapidly. Of that, likely half will be spent on EMR/EHR capabilities. US estimates of wide scale implementation of EMR/EHR range from $156 to $280 Billion in total investment over a 5 to 10 year period with between $16-48 Billion spent per year thereafter. Estimates of EMEA market potential developed for select target countries by ACN leadership suggest a cumulative $10 Billion opportunity for eHealth (EMR/EHR/Back-office) over the next five years, likely peaking in 2007-2009, with additional applications extending well beyond these dates. Likewise additional opportunities exist in other markets and countries.

There are many different suppliers to the healthcare market, and these include offerings from mainstream vendors, and also specialized, boutique providers to the health industry. Most current products are specific to a care domain, or provide an extremely "thin" layer of functionality across a care setting. Other products are specific to the underlying architecture and provide, typically, standards based interface to a vendor's business logic and architecture. There is a need for a truly flexible "middleware" architecture that is agnostic to underlying architectures, whilst being able to interface to both existing systems and newly developed technologies in a secure, robust manner protecting both the privacy of patients, and the integrity of the care process.

Healthcare technologies have expanded over the last 25 years typically in an organic manner. This has meant that technologies have developed at the same pace as healthcare, with associated advances in specific areas. To date, technology vendors have addressed their forays into healthcare through the interfacing with existing platforms, or the development of functionality that simply replicates that which is currently offered by competitive vendors. Innovation, such as it is, is led by clinical need rather than technological capability, and, treatment of the healthcare enterprise as a single domain has not occurred.

The healthcare market is a global one, whereas healthcare provision is by necessity regional, and there are few truly country (or nation) wide providers of healthcare. However, political motivations, based upon an understanding of technological potency, are driving the need for an enterprise approach to healthcare that pushes the boundaries of existing technologies. Indeed, the drive for a truly national healthcare record is one that will require extensive innovation and evolution of current technologies with novel approaches being required to seemingly intractable problems.

While other countries, including the United Kingdom and Australia, have attempted to create a national, centralized medical record repository, these efforts have met mixed results. Furthermore, the methods employed in other countries partially rely on state control of health facilities, thereby allowing systemic harmonization of health records which is not possible in the United States where the privately created health records may have varying formats and contents. Moreover these previous solutions could not be scaled to operate with the significantly larger number of medical records in the United States, the world's largest health care market.

To accomplish the goals of exchanging authorized, secure, timely, and accurate health information, many organizational, political and technical challenges need to be overcome. Specifically, a successful health information exchange should:
- address organizational issues such as privacy, security, data ownership, and change management, in addition to technical issues
- effectively merge patient records from multiple sources and avoid duplicate records that result in increased errors and decrease information availability
- support the ability to aggregate data from multiple, different clinical systems and EHRs within and across regional health communities by using common acquisition, interoperability, transformation and normalization processes
- have a security and access architecture to facilitate trust, auditing and cross-domain data access management
- have a robust data model and de-identification/re- identification capabilities that support data analysis and evidence-based health query capabilities
- be able to support the addition of new applications and transactional systems in a scalable and cost effective manner, and
- be flexible to support rapid program changes

By effectively addressing these issues, the delivery of health care can be dramatically improved.

US 2004/0083217 A1 discloses a method for collecting and distributing clinical data for data mining. The method comprises selecting a local report for collection, where the local report is a structured reporting object and includes a local clinical code, patient identification data and report collections status data. The local report is sanitized by removing the patient identification data. The local report collection status is updated to reflect the selecting. The local report is mapped to a common format report and the mapping includes: accessing a knowledge base that includes the local clinical code and a corresponding common format clinical code; and replacing the local clinical code with the corresponding common format code. The common format report is then transmitted to a data mining host system that includes a data repository.

WO 2005/088506 discloses systems and methods for gathering, utilizing, and/or distributing medical information. The systems include a means for receiving medical information from a plurality of sources. Such sources include a physician, a patient, and an implantable medical device. The systems further include a means for converting medical information from an implantable medical device to a selected format, and a means for distributing the medical information to one or more databases.

### SUMMARY OF THE INVENTION

The scope of the invention is defined by the appended claims.

The solution described herein documents a novel approach to building and deploying a technology platform that enables the exchange of semantically normalized data between different health care institutions. Embodiments of the present invention provide a system and related method for seamlessly linking a plurality of disparate, remote health records sources to enable the real-time collection, processing and centralized storage of health records, along with enabling controlled access to the centralized storage. This platform can be used to support clinical care at the regional or national level, public health surveillance, clinical trials, drug monitoring, care management initiatives, ePrescriptions, and other health care processes.

The interoperable healthcare data exchange platform seamlessly links a plurality of disparate, remote applications generally representing provider systems containing electronic health records (EHRs) to enable the real-time collection, processing and centralized storage of health records at a data store, along with enabling controlled access to the centralized storage.

The central health records data store receives, in real time, substantially complete messages containing electronic medical data input from multiple, disparate providers and sources of health records. Embodiments of the platform enable semantic normalization of the health records by converting the data in the health records to standardized message formats using a data conversion engine and mapping the converted data to standard terminologies (such as FHA terminology standards) using mapping products. This data store may be located at any convenient place and provides storage as well as programmatic contributions to the operation of the system.

In embodiments of the present invention, the platform provides security and privacy of protected health information through an integrated approach that provides a workable way to obtain sensitive patient information and to facilitate control by the patient to her information for all healthcare purposes, not solely direct clinical care. The platform is flexible and scalable to provide interoperable security and access architecture as need to provide auditing and cross domain access management, as well as role-based access control that provides for different organizational structures, privilege authorization, as well as patient-provider relationships which reflect the direct care relationship between a patient and their care providers (or groups of providers) including, the wider health and social care team, and also facilitating patients to specify those who they wish to have access to their care record.

### BRIEF DESCRIPTION OF THE DRAWINGS

A more complete understanding of the present invention and advantages thereof may be acquired by referring to the following description taken in conjunction with the accompanying drawings in which like reference numbers indicate like features, and wherein:
FIGS. 1A and 2-3 are a high-level schematic diagram of an interoperable healthcare data exchange platform accordance with embodiments of the present invention;
FIG. 1B depicts high-level schematic diagram of a secure data output system for the an interoperable healthcare data exchange platform of FIGS. 1A and 2-3 in accordance with embodiments of the present invention;
FIGS. 4-7 are a high-level schematic diagram of an interoperable healthcare data exchange platform in accordance with embodiments of the present invention; and

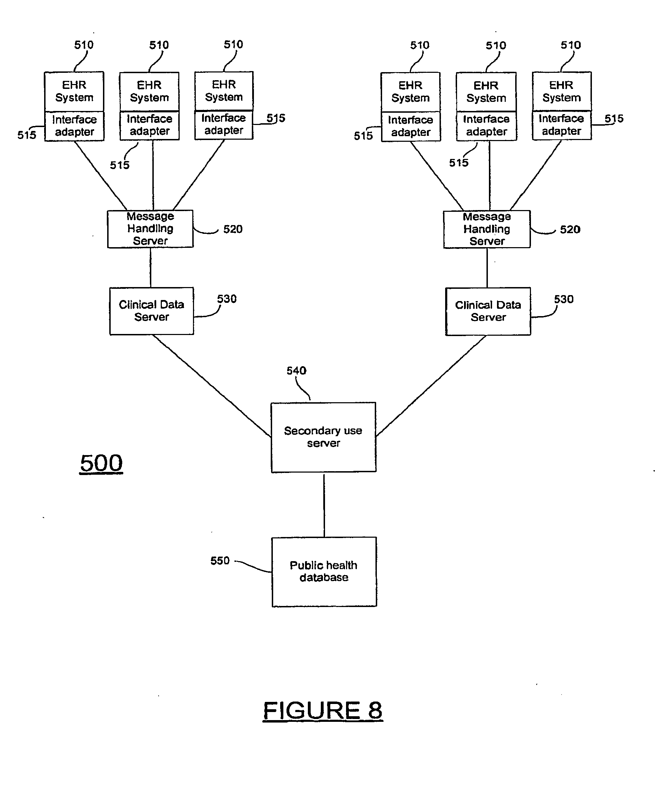

FIG. 8 is a schematic diagram of a health data collection network in accordance with embodiments of the present invention.

### DETAILED DESCRIPTION OF THE PREFERRED EMBODIMENTS

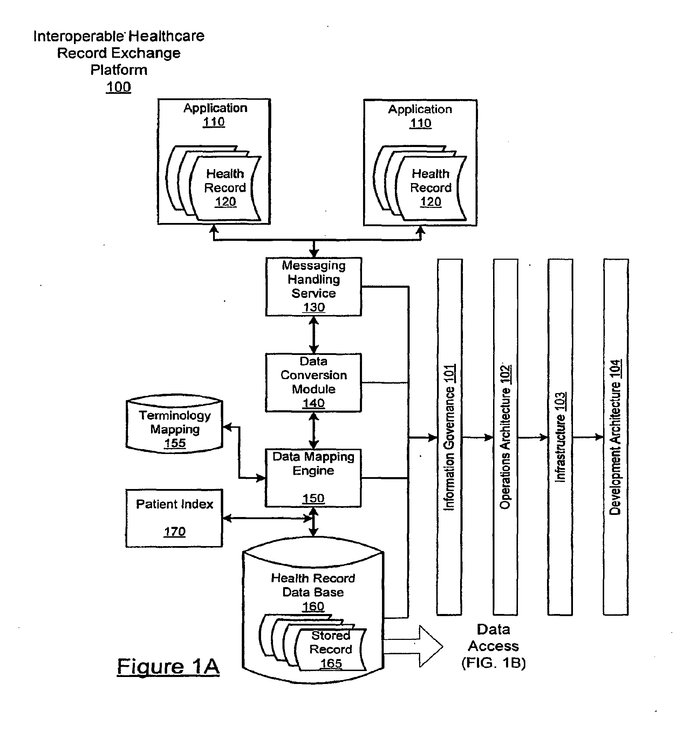

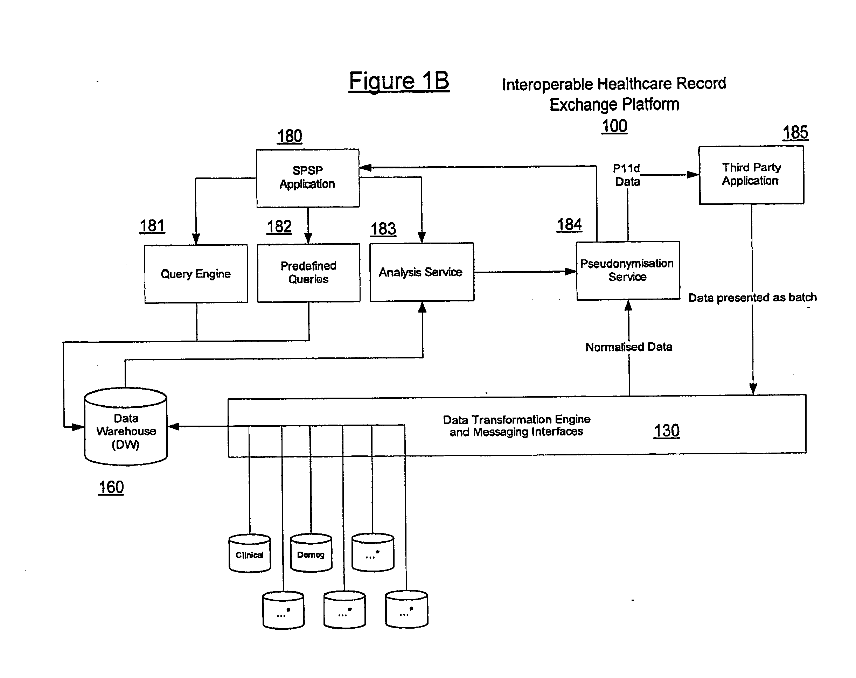

FIG. 1 depicts an interoperable healthcare data exchange network 500 of the present invention and its component parts. Specifically, the network 500. As described in greater detail below, the network 500 collects health care data from multiple disparate electronic health records (EHR) systems 510 at various locations. The EHRs, for example, may be associated with doctors, hospitals, other treatment centers, insurance companies, etc. Consequently, the various EHR systems 510 store the EHR in different formats and protocols. In response, in the network 500, each of the EHR systems 510 has an associated interface adapter 515 for preparing and standardizing the EHR for transmission to a messaging handling server 520. For example, interface adapter 515 may adapt the EHRs to Health Language 7 (HL7), version 3, a standardized XML schema for the transmission and processing of health records, described in greater detail below. The a messaging handling server 520 receives the standardized EHRs and forwards the standardized EHRs to regional clinical data servers 530, using advanced database techniques to store and organize the standardized EHRs to allow the stored, standardized EHRs to be accessed and processed as needed to achieve desired results, for example through a secondary use server 540 that is programmed, as described below to extract meaningful data. Similarly, a centralized public health records 550 may aggregate the health records from the various clinical data servers 530. These and other aspects of the present invention are described in greater detail below.

FIGS. 1A-B and 2-3 comprises a general overview of the interoperable healthcare data exchange platform 100 of the present invention and its component parts. The interoperable healthcare data exchange platform 100 seamlessly links a plurality of disparate, remote General Practice (GP) applications 110 generally representing provider systems containing electronic health records (EHRs) 120 to enable the real-time collection, processing and centralized storage of health records at a data store 160, along with enabling controlled access to the centralized storage.

As described in greater detail below, the platform 100 provides novel methods and software to partition and manage data, to maintain performance, and to protect the integrity of the data. The platform 100 may include, for example, information governance software 101 control access to stored data 165 and to verify the accurate transfer and storage of medical data 120; operations architecture 102 and infrastructure 103 to reliably implement the harmonizing data conversion and mapping, storage, and access; and development architecture 104 to allow users to implement and modify the platform 100 as needed.

Briefly, the central health records data store 160 receives, in real time, messages containing electronic medical data 120 input from multiple of general purpose (GP) applications 110 representing various, disparate providers and sources of health records 120. As described in greater detail below, embodiments of the platform 100 enable semantic normalization of the health records 120 by converting the data in the health records 120 to standardized message formats using a data conversion engine 130 and mapping the converted data to standard terminologies (FHA terminology standards) using mapping products 150.

In this way, the platform 100 optionally provides a data capture workflow to capture data at the provider location (represented by the GP applications 110) using tools and workflow with structured data to allow input of relevant demographic or clinical data for patients. For example, the platform 100 may allow providers to input relevant clinical data using their existing electronic medical records (EMR) systems. Typically, a provider enters data 120 into the GP application 110. This entry is documented in their system as an "episode," and the episode generates a message to an associated data store 160 such as a RHIO. Using the techniques described herein, an extract of the information added may be sent to the home RHIO and stored. Specifically, as described below, the platform 100 sends the episode data through various interface engines 130 to normalize the data 120 and integrate this captured data 120 into the repository 160.

In embodiments of the present invention, the data store 160 may comprise a series of linked servers or groups of separate servers, each of which is allocated to received health records for storage and access from a finite or defined group of patients, e.g., all patients within a certain range of social security numbers; all patients from a geographic region, etc.

The data store 160 may be located at any convenient place and provides memory as well as programmatic contributions to the operation of the system. Also, there may be multiple data stores 160. For example, in one implementation of the present invention, a data store is associated with a RHIO such that health records 120 within a region are collected by that data store 160. Then multiple data stores 160, each associated with a different RHIO, may combine to provide medical records 120 to a master data store 160 at a national health record repository at a National Health Information Network (NHIN).

It should be appreciated that a significant number of GP applications 110 may thus be involved in the platform 100. The platform 100 does not typically limit the number of providers that may communicate with and submit health records on a patient-by-patient basis to the health record data store 160. As described in greater detail below, each one of the GP applications 110 will provide unique medical information to the data store 160 through a network such as the Internet or a dedicated network or other network means.

The GP application 110 may constitute a health provider's or hospital's patient records that may include, for example, demographic and biographical data about the patients, a listing of each patient's symptoms; and another listing of the various courses of treatment administered to each of the patients, including the various prescribed procedures, tests, and drugs; a listing the doctors treating the patients. Similarly, the GP application 110 may be a combination of various general purpose applications used at the hospital, such as a combination of (1) a patient intake tool to acquire the biographical data and a listing of each patient's symptoms; and (2) a hospital billing program that tracks the courses of treatment administered to each of the patients and a listing the doctors treating the patients.

Furthermore, it should be appreciated that health records 120 may come from different GP applications 110 operating at different locations. Other examples of GP applications 110 that provide the health records 120 may include medical testing data software at a medical lab, prescription dispensing data software at a pharmacy, account and payment software from a public health care agency or an insurance company.

Thus, it should be appreciated that the patent records 120 obtained from the GP applications 110 may be quite complex and large. Also, since the patent records 120 come from 120 may be wildly different in format, style, and content. One may readily appreciate that health records 120 from GP applications 110 at different providers may be drastically different in format and content, let alone health records 120 from different GP applications 110 such as medical billing and medical testing software. Thus, aspects of the platform 100 entail converting the various health records 120 into a common format, and then processing the normalized health records 120 to allow meaningful access and use of the health records 120. For example, the platform 100 may allow an emergency room health care provider to access the data store 160 to acquire a new patient's stored health care records data 120 to better treat the patient.

The manner in which that information is provided to the central server 110 is now disclosed in greater detail. Referring back to FIGS. 1A and 2-3, a message handling service 130 regulates the transfer of the health records 120 between the provider GP application 110 and the data store 160. One example of a commercially available message handling service is Cloverleaf by Quovadx of Greenwood Village, CO.

The message handling service 130 administers the detection and forwarding of the new health records 120 from the GP application 110 to the data store 160. For example, the message handling service 130 may detect new health records 120 at the GP application 110, and then forwards a request to have the GP application 110 forward the new health record 120. Likewise, to better manage the production environment, the message handling service 130 may allow users to monitor message flow and to define proactive alerts. In embodiments of the present invention, the message handling service 130 is recoverable and can be configured to save a copy of a message in an internal work queue, database or file so that message are not lost.

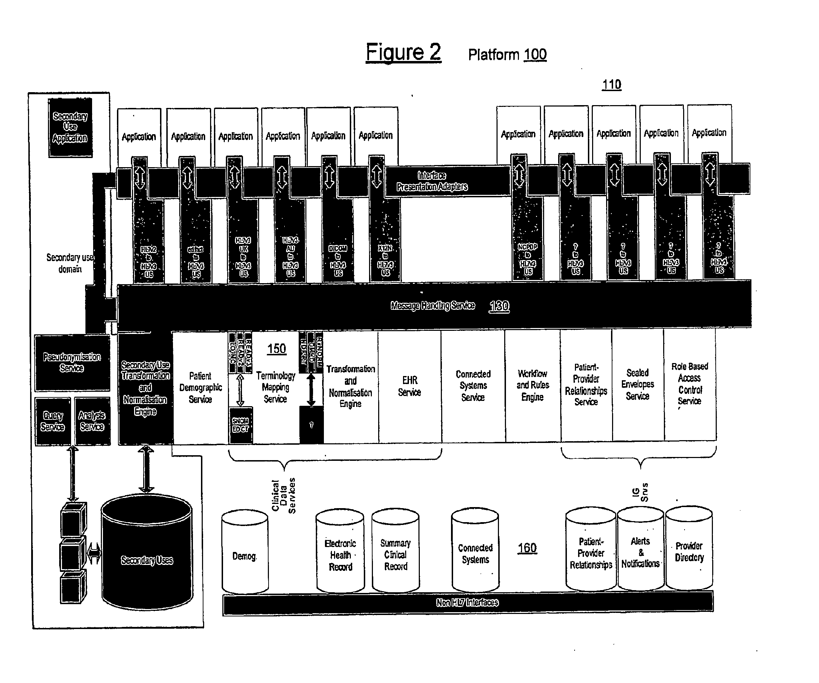

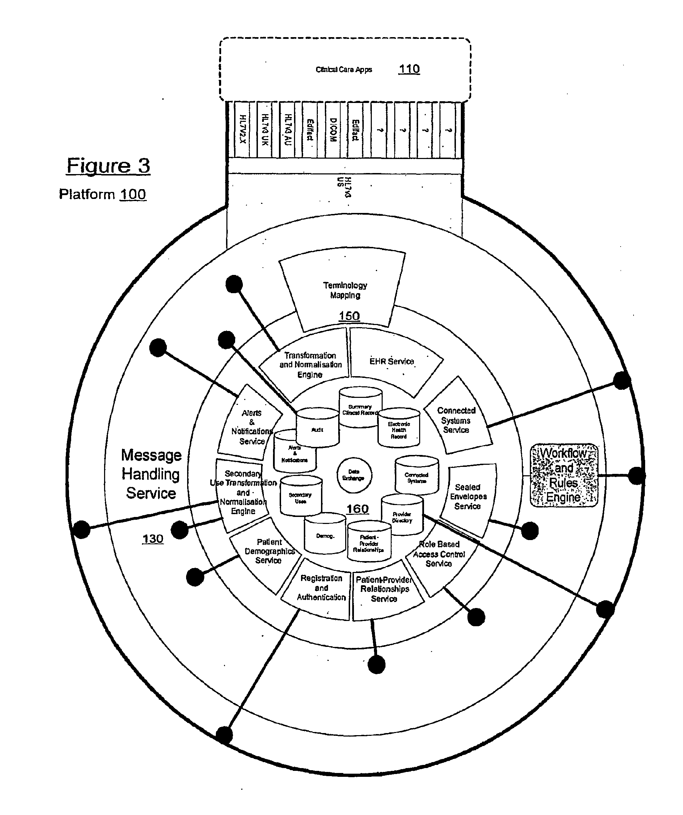

One aspect of the message handling service 130 may reside at the provider location with the GP application 110. As described above, the GP application 110 typically stores the health records 120 in various formats. In other words, each of the GP applications 110 natively stores and transmits its data records 120 in a different format. In response to this problem, the message handling service 130 enabled powerful application level integration using a library of application integration adapters, where each of the GP applications 110 has one or more appropriate associated adapters to transform transmitted health records 120 into a standard message format for data transfer such as extended meta language (XML) or Health Level 7 (HL7). In this way, the message handling service 130 is an interface to allow for automatic import of health records into centralized patient records at the data store 160 via standard protocols (HL7, XML). For example, FIGS. 2 and 3 suggest data conversion to HL7v3 (United Stated version) or other future versions of HL7 from various other known formats such as HL7v2, Edifact, HL7v3 (United Kingdom Version), HL7v3 (Australian Version), Dicom, X12N, NCPDP.

In this way, the platform 100 enables the extraction of data from multiple disparate sources in the healthcare value chain (such as Providers, Commissioners, Payors, Laboratories and Pharmacy Benefit Managers (PBMs)), agnostic to input systems, "at scale", and regardless of terminology codes used or geography, via interfacing engines.

The term "standardized output" has been used to describe the output of an ontology application implemented in the ontology processing block. This term should be read as encompassing any output form acceptable to an external system, whether human or machine. For example, in the context of the healthcare billing code application, there are many standards that might serve to define the exact nature and content of the system's output, including standards established by the Health Insurance Portability & Accountability Act (HIPAA), International Classification of Diseases Clinical Modification (e.g., ICD-9-CM; ICD-10-CM, etc.), Health Care Common Procedure Coding System (HCPCS) (e.g., HCPCS Level II codes), Current Procedural Terminology (CPT-Y) (e.g., CPT-4; CPT-5, etc.), Health Care Financing Administration (HCFA), Food & Drug Administration (FDA), Veterans Affairs (VA), Department of Health and Human Services (HHS), Centers for Medicare and Medicaid Services (CMS), National Library of Medicine (NLM), and the World Health Organization (WHO), etc.

The term "standard" or "standardized" in the foregoing context may have reference to either the content and/or the form factor of the resulting output. That is, the standardized output might not only include properly identified and related healthcare billing codes, but it may also be presented in a form ready for immediate consumption by downstream systems (e.g., be interface ready via XML - such as HL7, etc.).

In a preferred implementation of the present invention, these interface engines within the message handling service 130 will convert the local health record 120 into standardized HL7 message formats for transfer to the data store 160. Electronic medical information is often exchanged using well-known HL7 data encoding, and information from medical institutions may be obtained electronically by interpreting HL7 communications among hospitals, insurance companies and pharmacies, following transformation from the native format of the data records 120.

The coding used in HL7 data typically includes IDC codes for diagnostic information, CPT codes for treatment information, and FDA data for medications. Each portion of the information taken individually only infers to a particular medical situation. Taken together, however, the patient's record may be precisely updated. This information can include treatments and medications of all types, even those not officially approved for certain conditions.

To further complicate the data transform needed to forward meaningful health records from the GP applications 110 to the data store 160, different versions of the standard messaging protocols exist, and the filters in the message handling service 130 may not produce the correct version as needed for an interoperable platform 100.

For example, the HL7 Version 3 Reference Information Model (RIM) was recently completed. HL7 Version 3 is a definitive HL7 messaging standard, incorporating more trigger events and message formats than any previous version. Version 3 uses the RIM, as described below as a common source for the information content of specifications. As part of Version 3, HL7 specifications draw upon codes and vocabularies from a variety of sources. The V3 vocabulary helps to assure that the systems implementing HL7 specifications have an unambiguous understanding of the code sources, value domains and semantics that they are using.

HL7 Version 3 is different from previous versions, such as Version 2 that is commonly used in most current message handling services 130. New capabilities offered in Version 3 include:
- Top-down message development emphasizing reuse across multiple contexts and semantic interoperability;
- Representation of complex relationships;
- Formalisms for vocabulary support;
- Support for large scale integration;
- Solving re-use and interoperability across multiple domain contexts;
- A uniform set of models;
- Expanded scope to include community medicine, epidemiology, veterinary medicine, clinical genomics, security, etc.

The Reference Information Model (RIM) is the cornerstone of HL7 Version 3, described in greater detail below. An object model created as part of the Version 3 methodology, the RIM is a large pictorial representation of the clinical data (domains) and identifies the life cycle of events that a message or groups of related messages will carry. HL7 RIM is a shared model between all the domains and, as such, is the model from which all domains create their messages. By explicitly representing the connections that exist between the information carried in the fields of HL7 messages, the RIM increases precision and reduces implementation costs.

It is known to convert from HL7 version 2 to version 3. For example, please refer to U.S. Published Application No. 20060161840.

Similarly, an HL7 template is a data structure, based on the HL7 Reference Information Model, and which expresses the data content needed in a specific clinical or administrative context. They are prescribed patterns by which multiple OBX segments may be combined to describe selected, gross observations. Some observations may be quite simple, such as blood pressure readings that involve a set of expected observations (i.e., systolic, diastolic, patient position, method, etc.). Other more elaborate diagnostic procedures may involve hundreds of related pieces of information, including anatomy, orientation, sequences of measurements, etc. Templates provide a means of coupling the multiple OBX segments needed to send the observation with separately encapsulated rules for combining/validating them for the particular observation. Based on user need and preference, the template offers the user the advantage of defining the collection of OBX segments needed and the corresponding set of validation rules once, and once, defined, the structure can be used again and again. Since they are based on a specific user's needs/requirements, Templates can be plug-and-play at a given user site. The HL7 template are composed of data structures drawn from the HL7 RIM, that make use of HL7 vocabulary domains.

HL7 Version 3 further includes a coded vocabulary that, when used in conjunction with HL7 and related standards, will enable the exchange of clinical data and information so that sending and receiving systems have a shared, well defined, and unambiguous knowledge of the meaning of the data transferred. The purpose of the exchange of clinical data includes but is not limited to: provision of clinical care, support of clinical and administrative research, execution of automated transaction oriented decision logic (medical logic modules), support of outcomes research, support of clinical trials, and to support data reporting to government and other authorized third parties.

While the foregoing discussion exclusively discusses the use of HL7, other comparable health record communication protocols are known and may be similarly employed by a message handling service 130.

As described above, a message handling service 130 may not output the desired new protocol. Accordingly, embodiments of the present invention may further include an optional data conversion module 140 to perform the non-trivial task of converting messages in a first standard protocol to a second desired message protocol. For example, as laid out above, there are significant differences between HL7 versions 2 and 3.

Returning back to FIGS. 1A and 2-3, the platform 100 further includes a data mapping engine 150 that stores terminology mapping data 155 that may be used to support health data collection and organization data by the platform 100. In this way, the health data records 120, message normalization by the message handling service 130, may be normalized from terminology perspective. The data in the messages that are sent data store 160 is preferably mapped to standard Federal Health Architecture (FHA) terminologies (e.g., SNOMED CT, LOINC, or other such hierarchy). The mapping engine 150 has information about the relevant terminology hierarchy and maintains information about the relationship between terms of the hierarchy. The data mapping engine 150 may comprise a module hosted separately from the platform 100. Several commercial vocabulary services are available, for example, from Apelon Inc. of Ridgefield, CT.

Preferably, embodiments of the data mapping engine 150 support terminologies that conform to a common standard such as SNOMED-CT (Systematized Nomenclature of MedicineClinical Terms). SNOMED CT is a dynamic, scientifically validated clinical reference terminology that makes health care knowledge more usable and accessible. The SNOMED CT core terminology provides a common language that enables a consistent way of capturing, sharing and aggregating health data across specialties and sites of care. Among the applications for SNOMED CT are electronic medical records, ICU monitoring, clinical decision support, medical research studies, clinical trials, computerized physician order entry, disease surveillance, and image indexing and consumer (patient) health information services. The Core terminology Core content includes the technical specification of SNOMED CT and fully integrated multi-specialty clinical content, and the Core content includes the concepts table, descriptions table, relationships table, history table, an ICD-X-CM mapping, and the Technical Reference Guide. SNOMED CT further includes offers content, tools and services that build upon the Core content. For example, the SNOMED CT US Drug Extension includes proprietary medications approved for use in the United States. It links to the Core for electronic prescribing and clinical decision support which also relies on terminology for drug allergies, contraindications, drug-induced diseases, and lab tests used to monitor drugs. Currently, the Core terminology contains hundreds of thousands of health care concepts with unique meanings and formal logic-based definitions organized into hierarchies. Currently, the fully populated table with unique descriptions for each concept contains more than a million descriptions, and millions of semantic relationships exist to enable reliability and consistency of data retrieval.

In one embodiment, the data mapping engine 150 can be regularly updated as new terminology and other data becomes public. For example, updates of SNOMED CT are released twice a year. Alternatively, the terminology mappings 155 may be manually updated as needed for preferred operation of platform 100.

The terminology, including vocabularies, code sets, and identifiers, is employed in characterizing or identifying a health provider organization, a location in an organization, a healthcare worker, a medical condition, a health service, a cost of a medical procedure or service, a payer organization, or a particular health plan. In this way, the health data store 160 may contain medical terms, vocabularies and identifiers in addition to organizational characteristics, as well as location and other information. A medical code set as used herein is any set of codes used for encoding data elements, such as tables of terms, medical concepts, medical diagnosis codes, or medical procedure codes.

Proper categorization of a data record 120 may require, for example, a determination of whether the patient has diabetes. In order to make this determination, the platform 100 sends a request to the data mapping engine 150 for all of the various sub-types of diabetes. These subtypes are also known as the "subsumed terms" associated with diabetes or the set of unique medical IDs subsumed within the term "diabetes." In response to receiving the parent identifier of "diabetes," data mapping engine 150 returns the set of subsumed terms. Using these subsumed terms, data mapping engine 150 can query the terminology mapping 155 for the full complement of possible types of diabetes. The terminology contains unique identifiers for each medical element and hierarchical terminologies for each element. For example, a medical term such as "Type II Diabetes" may subsume many types of diabetes such as Type II diabetes with renal complications, and insulin resistant Type II diabetes. If the guideline has a criterion that asks, "Does this patient have Type II Diabetes?" the encoding would ask "Does this patient have SNOMED CT: 44054006" where SNOMED CT: 44054006 is the code for Type II Diabetes. When the terminology mapping 155 is queried for subsumption with "SNOMED CT: 44054006", it would respond with the relevant subsumed meanings of "Type II Diabetes".

Continuing with FIGS. 1A and 2-3, the data store 160 receives and stores modified data records 165 following conversion by the message handling service 130 and conversion module 140 and following data mapping by the data mapping engine 150. The updating preferably includes algorithms to manage version skewing in terminology as terminology changes over time. For example, the existing records 165 may be remapped by the mapping engine 150 using updated terminology 155 so that the existing records 165 may be integrated with incoming, newly mapped data records 120.

Preferably, embodiments of the present invention provide methodology and software as needed to integrate the terminology normalized data from the mapping engine 150 from a commercially available data mapping engine as needed by the data repository 160.

While data mapping techniques are known, it should be appreciated that the platform 100 provides methodology and software to provide the speed to get the data into the data store repository 160 at a sufficiently large scale.

Returning now to FIGS. 1A and 2-3, the data store 160 receives the data records after protocol conversion and data mapping and stores these converted, mapped health records 165. The data store 160 partitions and manages the data as needed to maintain performance and protect the data 165. Specifically, the converted, mapped health records 165 are used to populate a centralized repository 160. Thus, the data store preferable supports the clinical data structures of the normalized records 165. As described in greater detail below, the data store 160 entails databases, data models and methodologies that provide decision support, privacy services, aggregated views, and other desirable features. In this way, the normalized data records 165 will be available for multiple secondary uses as well as potentially provide information to a treating physician.

Data 165 held within the data store 160, although normalized, should be a sufficient richness and quantity to subsequently enable robust, meaningful analyses. A non-exhaustive list of possible analyses include:
- Data linkage across patients;
- Data linkage across care episodes;
- Longitudinal analysis of patient care;
- Age based analyses;
- Gender based analyses;
- Condition based analyses;
- Geographic Analyses;
- Epidemiological analyses;
- Bio-surveillance; and
- Screening.

In one preferred embodiment of the present invention, that data store 160 receives and stores the normalized data records to provide an extended, distributed data model that is based on the HL7 Version 3.0 Reference Information Model (RIM), as described above. In this way, data accessed from the data store 160, such as messages that are sent to the regional data model, will comply with standard Federal Health Architecture (FHA) terminologies (i.e., SNOMED CT, LOINC, etc.) and a centralized repository shall be capable of supporting such vocabularies.

Normalization enables the creation of valuable data stores 160 that can be used for epidemiology studies, bioterrorism detection activities, quality assurance, cost containment, and clinical research. In addition, appropriately identified and indexed data 165 can be used to generate alerts for error reduction and to support disease management system development. Additionally, this same normalization process has the valuable by-product of supporting a consent management system that provides a workable way to sequester sensitive patient information and to allow for access control by the patient to his/her information for all purposes, not just direct clinical care.

In embodiments of the present invention, data store 160 is specifically designed for health care, and has a data model based on a health care data standard, such as the above-described HL7 Version 3.0 Reference Information Model (RIM). The data store 160 further supports a master patient index 170 (described in greater detail below) and federated security architecture at the NHIN and RHC level. For example, the data store 160 may be based on the Health Care Transactions Base (HTB) software marketed by Oracle® of Redwood Shores, CA.

In embodiments of the platform 100, the data store 160 may optionally include has several core components such a master patient index 170, a provider directory or a record locator service. In this way, the stored normalized data may be search by patient, provider, or other used defined criteria. For example, health records related to a particular condition, demographic, and course of treatment may be collected to aid in the evaluation of the efficacy of the specified course of treat for the specified demographic group.

For efficiency purposes, the data store 160 may optionally contain no identifiable personal information (i.e., patient name and address), but should contain coded information with a specified identifier for each patient. Patient demographic information is then externalized in the patient directory 170 in a high availability database or directory that is separate from the data store 160.

The patient directory 170, sometimes called Enterprise Master Patient Index (EMPI), optimally, includes a strict change control process for patient information to link disparate information of patients received from external systems into one file for the patient to avoid duplicate records. The patient index 170 may further enable complexity and architecture that is not available in current health record databases. For example, patient index 170 may allow the platform 100 to perform medical records management (using services, processes and building tools) across provider organizations. For example, maintenance may be initiated where a search for records associated with a person cannot find any relevant records. In other embodiments, the patient directory 170 may be implemented through novel approaches to secure patient privacy while still permitting appropriate tracing of patients by providers. Toward this goal, the platform 100 may include processes and software to coordinate across distinct provider organizations.

Accordingly, another embodiment of the present invention may include a provider directory (not illustrated). For example, the provider directory may be a multifaceted to allow a provider to be placed within a clinical hierarchical context. Moreover, a provider may operate on several different hierarchies. Thus, the platform 100 may include novel processes around the implementation and provide a provider directory for a national information network. However, the addition of a provider directory adds complexity by requiring management of other types of dynamic data in additional to patients

Referring to FIG. 1A, the platform 100 further comprises information governance 101 to provide security and privacy of protected health information. Aspects of the information governance 101 include an integrated approach that provides a workable way to obtain sensitive patient information and to facilitate control by the patient to his/her information for all healthcare purposes, not solely direct clinical care. The information governance 101 is flexible to allow customized access control rules as needed by various access functions, as described below. Specifically, the information governance 101 is flexible and scalable to provide interoperable security and access architecture as need to provide auditing and cross-domain access management. Thus, aspects of the present invention provide an approach to role-based access control that provides for different organizational structures, privilege authorization, as well Patient-Provider relationships which reflect the direct care relationship between a patient and their care providers (or groups of providers) including, the wider health and social care team, and also facilitating the ability of patients to specify those who they wish to have access to their care record.

The information governance 101 further includes an access control framework that can operate within either a hierarchical or federated model, and support the use of public/private key technologies (such as Public Key Infrastructure or PKI) where appropriate. The information governance 101 may further include (1) processes, policies and procedures for the robust identification, registration and issuance of access credentials to care professionals; and (2) processes, policies and procedures for the management, including key management, of all aspects of the security and confidentiality model - with such processes, policies and procedures to be harmonized with the care environment. Accordingly, the information governance 101 may provide the capability to aggregate data for a variety of cohorts in a pseudonymised or anonymised fashion, yet maintain sufficient controls to enable direct patient contact if required. Likewise, the information governance 101 may give the capability for a patient, subject to strict access controls, to be able to access their own health record formed from an aggregation of many different data sources across a variety of provider organizations, including the provision of software to enable patient access to health records

The information governance 101 may possible further allow patients to select specific parts of their record and place access controls upon them which are independent of those other access controls routinely provided. Likewise, the information governance 101 may provide care providers with the capability to select specific parts of the patient record which require special access controls to be placed upon them, independent of those other access control routinely provided

Referring now the FIG. 1B, the platform allows access to the data records 165 stored at the data store 160 in a variety of methods, depending on the granted level of access. For example, an authorized user, such as health care providers may be able to use the messaging system 130 to access the normalized data records 165 in whole. Other users may access particulars portions of the data records 165 after the data records 165 are stripped of confidential information. Other users may receive data created through analysis of the data records 165, such as selected statistical summaries.

For example, the application programming interfaces (APIs) may be integrated into data store 160 allow the development of applications that can leverage the semantically normalized data existing to support a wide variety of existing and future health care processes. Additionally, the normalized data can be exported into data warehouses for further analysis.

A portal with a graphical user interface may be available for patient and provider access and this same interface can be exposed as a web service to other applications. Additionally, the data can be leveraged by other applications through the development of web services that again are accessed through the above-described APIs.

As described above, the application programming interfaces (APIs) integrate to applications that can leverage semantically normalized data existing at the regional level or at individual health provider, research or pharmaceutical and biotechnology companies to support a wide variety of health care processes.

For example, for presentation of medical summary data, the platform 100 may include a custom portal (not depicted) with a graphical user interface which can be exposed as a web interface to providers and patients. In this way, a patient could potentially acquire information on the quality of a provider or a course of treatment. Alternatively, an existing application may be modified to access the data store 160 to acquire the summery. In this way, a provider could likewise acquire statistical information about course of treatment. Other more sophisticated user interfaces may be added to the data store 160 this purpose to allow intrinsic access to certain data. Then, a user may define search criteria to pull the desired medical summary information, and the requested data is brought into the presentation layer.

Referring back to FIG. 1B, the platform 100 enables Service for the Provision of Secondary Purpose data (SPSP) to provide de-identified, anonymized data through the data store, or warehouse 160. In this way, the platform develop a more innovative approach to the current, standard mechanisms including the ability to re-identify the data for patient identification and contact and other novel capabilities to instantiate privacy and security rules to enable desired secure access.

In the embodiment of the platform 100 depicted in FIG. 1B, the SPSP Application 180 is a direct user interface available as a web-browser based client. This UI enables control of all the querying, analytical and outputting functions through a server side application. Third party applications interact with the SPSP through messaging, and the provision of batch data.

Continuing with FIG. 1B, Data Transformation Engine and Messaging Interfaces, such as above-described message handling system 130 enables the. SPSP application and other third party applications to integrate with SPSP components. Multiple messaging interfaces may be supported, enabling data to be presented in a variety of formats. Also, as previously disclosed, data transformation services through the message handling system 130 are also provided to facilitate the normalization of data. This layer 130 typically does not provide semantic validation services, as all data is assumed to be valid prior to presentation, but may provide for the validation of data for out of range values, etc. Instead semantic validation services may be offered through other known tools. Alternatively, a different message handling service may be used as needed to format the desired output data.

Continuing with FIG. 1B, a variety of predefined queries 182 are provided through the platform 100 to allow users to undertake common and routine tasks. A query engine 181 may also be provided to enabling users to create ad-hoc or custom defined queries. An analysis service 183 enables manipulation of data. Data that is output from the SPSP typically passes through a Pseudonymization Service 184. This will pseudonymize the data dependent upon user access controls.

As described above, the data store 160 holds a normalized copy of all presented data. For purpose of data output, the data store 160 may also present a series of data marts (which might also be the result of a query).

In operation, the platform may provide two modes of operation, Batch, or Submission, mode where data is automatically forwarded and Interactive mode where the data forwarded upon user request.

Consequently, the platform 100 provides the ability to process patient identifiable data into pseudonymised data while preserving the ability to provide patient identifiable data. Moreover, the platform 100 provides a robust and flexible access control framework and automatic association of queries and analyses with the user performing such functions.

The platform 100 allows algorithms implemented within the SPSP 180 to be secure, robust, high performance, having low resource requirements, adjustable, and resulting in different products depending upon user, access control and function.

Continuing with FIG. 1B, the Pseudonymization Service 184 is generally capable of undertaking a variety of pseudonymization tasks, and the nature of these will depend upon user access controls and application function being performed. In essence, the platform 100 may output Patient Identifiable Data including non-coded information; Patient Identifiable Data using solely coded information; Patient Data in which any identifiers have been pseudonymized; and Patient Data in which all identifiers have been removed.

As the purposes to which output data will be put are many and varied, the platform 100 facilitates flexibility in the performed pseudonymization. Therefore, the platform is capable of re-running queries and analysis and outputting the results using a different pseudonymization regime than previously undertaken. Also, platform 100 allows analysis or query to be re-run using a defined "point in time," thereby enabling the same cohort to be output as was previously.

The Pseudonymization Service 184 further supports the linkage of data sets output. This is done in a manner that can be specific to the data, the user, a facet of the user role, or the purpose to which the data will be put, dependent upon user access controls.

In another embodiment of the platform 100, an optional record locater service (not illustrated) may be associated with the data store 160 or another database. The record locater service includes list of pointers telling where the original data records 120 is located (RHIO and/or Provider locations).

Embodiments of the present invention enable the operating and maintaining of a RHIO. The ability to normalize data and interface with a wide variety of systems results in an architecture that can scale and promote the sharing of health care data across a wide variety of provider organizations and systems. For example, the data store 160 may be operated by a service provider such as a Regional Health Information Organization (RHIO) which would initially be comprised of affiliated hospitals, staff, laboratories, physicians, care givers, intermediate and long term care providers and pharmacies. Each would have access to the RHIO operated central server and each would have an encryption code for its list of patients. That code would enable unidirectional input on a unique patient by patient basis to the central server, subject only to input programming criteria to insure clean data and limited bidirectional communication to confirm data receipt and to control the standardization of data, i.e., insure it is clean data. The encryption code would be secured through the patient hardware key plus the user name and password of the provider. This combination of code input would insure communication uniquely solely with the patient's record for input purposes only since the provider would not have the patient name and password. Rather, the combination of key code with provider name and password would limit the provider (via the server program software) to data input only in one preferred embodiment.

The RHIO would preferably create a backup record of all input data on a real time basis. That backup data record would replicate the RHIO data in case of system failure and would immediately be on line upon detection of a system failure. Such redundancy is a preferred feature of the system and would provide a source of information that could be separately used for research.

That is, certain fields of information could be rendered inaccessible for research purposes, such as name, address, social security number, etc. However, other fields of information could be made available for statistical research. Such research access could be subject to pre-access approval by the RHIO or other server operator and could also comprise an income source for the RHIO, e.g., payment for access to the medical information regarding the history of certain drug use. Again, the access to information would require patient permission that would be solicited and obtained upon assignment of and providing the patient with a key device, password, etc. All at the same time the patient would also likely provide various medical instructions such as a living will, organ donation information, emergency instructions, etc.

Emergency access by certain providers could be insured by a combination of the authorized providers key code and the patient name and/or password, and/or encryption key. For example, an emergency medical service (EMS) may have an encryption key device that, in combination with the device or password and/or name of an accident victim, provides access to that victim/patient's medical record. Thus, the patient/victim may have an RFID device, a USB device, or even a "smart card" which in combination with the EMS encryption key will permit access to the unique medical record of the patient/victim. The available information will typically, in such circumstances, comprise an emergency subset of patient information per a program that limits the information to the "need to know in emergency situation."

As can be seen, the system eliminates duplication of records, standardizes record keeping, permits access as needed, provides for contribution of information by multiple sources and most importantly is dependent upon patient participation.

The platform 100 of the present invention integrates both existing software assets and new technologies along with operating and execution processes to develop a technology platform, repeatable operational and development architecture and processes that support the ability to manage dynamic data from multiple types of stakeholders - patients, providers, etc. as well as aggregating data from multiple, different clinical systems and EHRs within and across health provider organizations, laboratories, health insurance organizations and/or governmental agencies, by using common acquisition, interoperability, transformation and transaction and medical terminology normalization processes.

In this way, the platform 100 effectively merges patient records from multiple sources and avoids duplicate records that result in increased errors and decrease information availability. The platform 100 further enables authorized, secure, timely, and accurate exchanges of semantically normalized health information among patients, clinicians, other providers and authorized entities. The platform 100 further provides a scalable security and access architecture to facilitate trust, auditing and cross-domain data access management that facilitates interoperability and/or federation. Overall the platform 100 provides a robust data model and de-identification/re-identification capabilities, supporting the secondary use of patient information (e.g. epidemiology studies, biosurveillance activities, screening, quality assurance, cost containment, clinical research and disease management) Secondary Uses means those uses that are not the provision of direct healthcare to a patient or group of patients. The Platform 100 may further optionally provide for the provision of analytical facilities to support secondary uses, as well as supporting a National Health Information Network (NHIN), Regional Health Information Organizations (RHIOs) and Regional Health Communities (RHCs) in exchanging information and be flexible for the growth and scalability of these initiatives.

Embodiments of the present invention provide readily available packaged solutions for most the architecture's technical components. Given the diverse and rapidly expanding landscape of possible technology solutions for the architecture, selecting the most appropriate solution for the architecture will require discussions and evaluation of the existing environment. Therefore, in this response potential vendors are mentioned but this is purely for indicative purpose and not a formal selection.

Moreover, the present invention provides innovative emerging solutions to provide high value for the architecture. For example, a networking solution, such as Cisco Application Oriented Networking (AON) may play a vital role in the proposed solution. The networking solution may be leveraged to develop a standard architecture layer for member institutions, the so-called Novel Integration Platform (NIP). The NIP can be rapidly deployed and will provide the advanced security and manageability required in the architecture's distributed architecture.

Furthermore, embodiments of the present invention can be adapted to include advanced security and privacy architecture since providing a secure environment that protects patient privacy is an integral component of the present invention. Security and privacy requirements are complex and will evolve over time, making packaged solutions insufficient to meet the architecture's security needs. The present invention may implement an advanced architecture for security and privacy protection, based on the experience gained during other significant clinical data exchange implementations.

Also, the operations architecture disclosed herein supports ongoing management, and the technical components outlined elsewhere make up the execution, or run-time, environment for the architecture. Based on experience, it is equally important to focus on another aspect of the solution - Operations Architecture. Operations Architecture is the set of capabilities necessary to monitor and manage the environment on an ongoing basis, once the solution is deployed. Given the importance of having the Operations Architecture ready when the solution is deployed, an approach to Operations Architecture is included below.

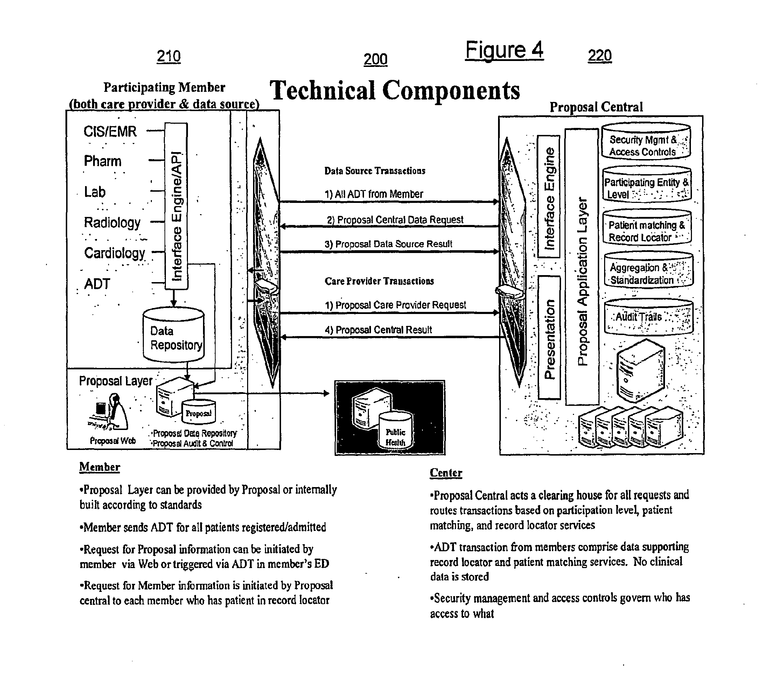

Referring now to FIG. 4, an interoperable health records platform 200 in accordance with another embodiment of the present invention is disclosed. As disclosed herein, the an interoperable health records platform 200 allows health records to be collected from participants 210 and collected at a central location 220.

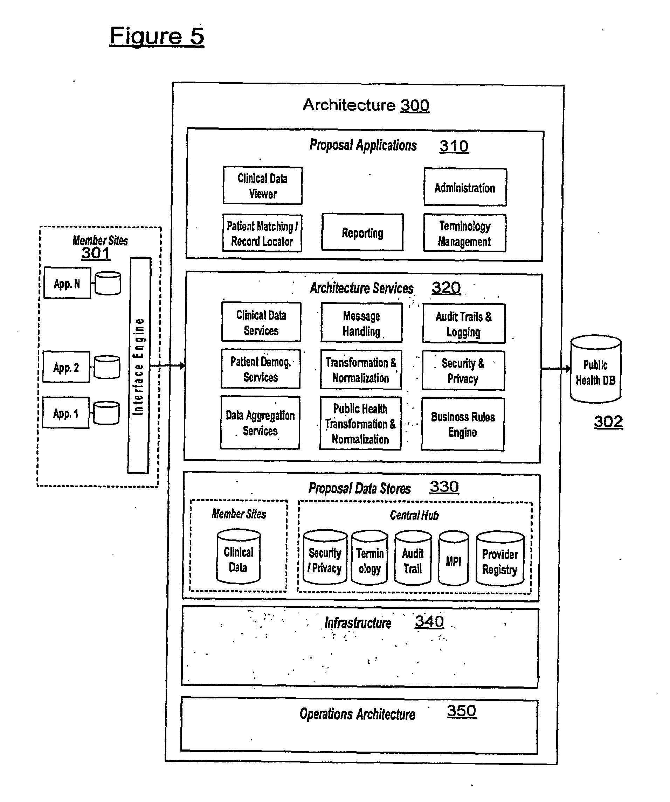

One would implement the technical components in a layered, modular fashion as depicted in platform architecture 300 in FIG. 5, primarily a logical view of the architecture.. The physical distribution of architecture components would be determined as needed. In making those physical distribution decisions, the invention may be adapted to ensure the most appropriate balance of security, functionality, performance and manageability.

The architecture will be able to begin by implementing only those components necessary to provide basic data exchange functionality. Once the basic data exchange functionality is in place, layering and modularity will also allow for incremental improvements to the architecture, as it minimizes the impact of changes to any one architecture component.

Each layer of the architecture 300 is now disclosed in greater detail. The applications 310 are in the layer that faces the end users. Applications allow the user to view or manipulate data in the architecture data stores. Applications may or may not take advantage of services. Architecture Services 320 is the layer that provides services to support clinical data exchange. These services are typically invoked by events, either user requests or other business events that trigger service execution. Data Stores 330 are Repositories that store data. Infrastructure 340 is the platform that provides infrastructure services. The Operations Architecture 350 is the set of capabilities necessary to monitor and manage the environment on an ongoing basis, once the invention solution is deployed..

The architecture 300 may further contain optional additional components (i.e., Integration Engine, Public Health Database) that will be described as necessary to provide full clarity on how one would implement the specific components of the architecture 300.

Continuing with FIG. 5, the Applications Layer 310 is the primary mechanism by which end users perform business functions. Exemplary applications within the application layer are given in Table 1.

| **Table 1** | | |
|---|---|---|
| **Application** | **Purpose** | **Used By** |
| Patient Matching / Record Locator | Match and locate patients based on demographic attributes. | ED Physicians |
| | | Other ED users |
| | | Other authorized users (e.g., receptionists) |
| Clinical Data Viewer | Provide access to clinical data captured in the architecture system. | ED Physicians |
| | | Other ED users |
| Reporting | Provide predefined reports and a mechanism for the definition of ad hoc data reports and analysis services. | based on reporting requirements; most likely outside the ED context |
| Terminology Management | Manage standard terminologies; allow users at each site to map standard terminologies to local terminologies. | the terminology managers at each member institution |
| Administration | Allow authorized individuals to perform administrative and maintenance activities, such as user management (enrollment, role, access control). | the administrative personnel (at each member institution) |

Again, it should be appreciated that Table 1 represents an indicative list of applications in the application layer 310, and should not be taken as definitive. For example, definition of an appropriate access control framework may be otherwise performed as needed.

As the Table 1 above indicates, physicians and other personnel within the Emergency Department (ED) will typically use two of the applications listed above: Patient Matching / Record Locator and the Clinical Data Viewer. Depending on the workflow that supports the architecture system, ED personnel may not even use the Patient Matching / Record Locator application.

The Architecture Services Layer 320 performs functions to support clinical data exchange between the members 301 as well as data transfer to the public health repository 302. Architecture services are described below in Table 2, along with their key functions. Each service has a well-defined purpose and will be deployed according to the present invention 's preferred implementation roadmap.

| **Table 2** | |
|---|---|
| **Service** | **Purpose** |
| Clinical Data Services | Process data both into and out of the clinical data repository: Capture data into the clinical data repository. |
| | Process requests from the hub and respond with clinical data. |
| Patient Demographic Services | Manage patient demographics data in the clinical data repository and the central master patient index. |
| Data Aggregation Services | Retrieve and aggregate data from member clinical data repositories. |
| Message Handling | Receive and parse incoming messages, invoke appropriate services based on the contents of the message. |
| Transformation & Normalization | Transform and normalize data from member institutions to enable sharing via the data exchange. For example, map local terminologies to the architecture standard terminologies. |
| Public Health Transformation & Normalization | Services to support uses such as age based analyses, gender based analyses, condition based analyses, geographic analyses, epidemiological analyses, biosurvelliance and screening. |
| Audit Trails & Logging | Services to log key data about events within the system. |
| Security & Privacy | Services to secure the application and data as well as protect patient privacy. |
| Business Rules Engine | Store rules that manage the execution of workflow. |

The Architecture Services layer 320 provides the core components that enable the data exchange to function. It is anticipated that some of these services will be physically deployed at the member sites 210 and some will be deployed at the central hub 220. The invention may be adapted to determine the precise details around physical distribution of components. It may be possible to manage the components that are physically deployed at the architecture member sites by developing a Novel Integration Platform (NIP) - a standard bundle of components (including data stores and potentially architecture services) that will be physically deployed at member sites. By standardizing the NIP, one can rapidly deploy the architecture to member sites. In addition, it is expected the NIP to leverage Cisco's AON solution, enhancing the architecture's ability to provide a secure and managed infrastructure at member sites.

The approach for integrating member data into the exchange is designed to ensure consistency within the NIP, minimize the burden on members and leverage member's existing investments in applications and integration tools. A standard set of messages that the NIP will accept and process may be defined. If source transactional systems at member sites can supply these messages, the NIP will accept them. This minimizes the burden on the member sites by reusing existing interfaces. If source transactional systems cannot supply these messages, members can utilize existing in-house interface engines to supply these messages, thus preserving their investment in these tools. If members do not have interface engines, the architecture will provide a recommended standard interface engine. Member institutions may need to configure or develop adapters for some applications in order to utilize the interface engine.

Due to the sensitivity of patient health data as well as established federal patient privacy regulations, security and the protection of patient privacy should be among the most important concerns when implementing a clinical data exchange. Given the ongoing national debate over the impact of health data exchange on patient privacy, it is possible to implement a number of advanced features to provide security and ensure the protection of patient privacy. Those features are described below. Specifically, a variety of security services may be offered in the Architecture Services layer 320, providing a holistic approach to the security and privacy of patient information. The techniques adopted are designed to provide a high level of integrity to both existing estate and newly deployed systems. The precise configuration of the security components will depend, but over and above the traditional techniques for ensuring privacy and confidentiality of information, the architecture 300 may further include augmented approaches for Registration and Authentication, Role Based Access Control (RBAC), Patient - Provider Relationships, seal envelopes, and Audit and Alerting.

For example, in the registration and authentication service is one that can implement differing levels of assurance in the credentialing process used to identify and register both healthcare users and healthcare systems, and depending upon organizational requirements can implement various different authentication mechanisms. All authentication may be based around a Public Key Infrastructure (PKI) - providing for strong authentication of users, and mutual authentication of all system end points. PKI comprises of a system of digital certificates, certificate authorities (CA), and other registration authorities that verify and authenticate the identity of each party involved in an Internet transaction. PKI components include maintaining root certificates, authentication, authorization, and encryption and decryption of transactions.

The architecture 300 enables regional data interchange among various healthcare hospitals and institutions. These institutions may not be directly affiliated and have their own information security policies and AAA (Access Control, Accounting, Audit), methods. The members are, for the most part, separate business entities with their own data centers, security designs, and management policies. Authentication amongst these separate entities suggests building a loosely coupled and federated identity management system, however, the precise details of this will depend upon the governance model implemented with the proposed solution. There may be cooperation with the members to ensure that this is robust, deployable and scalable. Support for digital signatures provides non-repudiation, thereby guaranteeing that a message or data can be proven to have originated from a specific person. Non repudiation will be supported where required by the architecture's needs and to meet legislative obligations.

A flexible, scalable, manageable approach to Role Based Access Control (RBAC) may also be desirable. This feature enables a layer of commonality to be placed across multiple provider organizations. RBAC constraints can be enacted at a variety of levels. Applications that are framework aware can use the privilege information offered by the framework to enact security constraints within the application itself. However, for those applications which are not framework aware, RBAC constraints can be implemented within the messaging layer(s), thus constraining the manner in which applications can interact with the architecture as a whole.

This architecture 300 is complimentary, yet orthogonal to that of Role Based Access Control, and facilitates the declaration of relationships between patients and their care providers. This relationship can then be used to constrain user access to solely those patients to whom the provider is providing care (and therefore has a relationship with). Triggers for the creation and termination of the relationships are implemented within applications that are aware of this framework. However, as for RBAC, this architecture 300 can also be implemented within the messaging layer(s), thus constraining access to data within applications that are not PPR capable. Unlike RBAC however, this framework requires a greater degree of management when an application is not PPR aware, as relationships can not be automatically created and terminated, therefore an additional management application is required in limited cases. The Patient-Provider Relationship can also be used independently of access control to represent the care team around a patient. A vocabulary for PPRs is implemented, which holds both access control semantics, and also broader care relationship semantics. Therefore, PPRs can be interrogated and yield the entire care family for a patient.

The sealed envelope has two facets - that of the Patient's Sealed Envelope, and also the Clinician's Sealed Envelope. The former can be used by the patient to place additional access controls upon their shared data. This enables the patient to restrict access to certain parts of their shared record and is of use when a patient has special privacy requirements. However, the Patient's Sealed Envelope is not intended for regular use, as the interaction of correctly implemented Role Based Access Control and Patient-Provider Relationships provide a sufficiently granular access control framework to prevent access to patient data by unauthorized individuals, who have no need to know. The Clinician's Sealed Envelope can be used by clinicians to place special access controls around specific sections of a patient's shared record. This is of use where specific diagnoses or information concerning prevalent conditions are deemed by the clinician to be potentially harmful to the patient, or need to be withheld from the patient for another reason. This is especially of use in areas such as Mental Health, and Genitourinary Medicine. Specific policies and procedures must be implemented by organizations around the use of sealed envelopes - concerning specifically the classes of information that can be placed within the envelope.

All systems deployed as part of the architecture may incorporate robust audit and analysis systems as standard. These will propagate audit information to a logically centralized audit repository, which shall be available for query and analysis by authorized users. Those packaged applications which are incorporated into the architecture may not be considered to have the same level of audit functionality as custom built systems. This is not to say that such systems have poor audit capabilities, simply that they may not have the same facilities as available in a custom-built system - and they are unlikely to have the integrated nature of audit subsystem available that is being proposed. Therefore, where appropriate, auditing of messaging output from systems will be implemented - thus facilitating an integrated audit facility inclusive of all platforms. As explained above, a networking solution, such as AON® can play a vital role in the architecture's audit and alerting approach. Acting as a network interception point for application message traffic, each security gateway node within the sites can be configured to act as a sensor that can capture, process, and log highly granular information about application messages. An administrative user interface will be available for the security officers from member organizations to manage such audit trails. Additional intelligence in logging transactions is provided in a networking solution's ability to generate and route events external systems based on message content inspection. This can be very useful in tracking patient opt-outs, which can be appropriately tagged in the security system such that access will be denied to patient information for those patients who have opted-out of the program.

Returning now to FIG. 5, the Data Stores Layer 330 includes various data stores that could be implemented as part of the proposed solution, as depicted in Table 3. It is anticipate that the number and/or nature of these data stores will change as the solution design evolves. The structure of several of these data stores may be driven by the packaged solution that the architecture selects. Therefore, this section provides an overview of the likely data stores, along with their purpose and how they would be most frequently accessed.

| **Table 3** | | |
|---|---|---|
| **Data Store** | **Purpose** | **Accessed By** |
| Member Clinical Data Repository | Stores patient demographics and clinical data. May also store audit trail data. The data repository will be structured to allow the persistence of data in a standardized fashion, while still permitting each member to implement different data types at different times. | Clinical Data Services |
| Master Patient Index (MPI) | Stores patient demographic information along with any attributes necessary for matching and/or locating patients. | Patient Demographics Service Patient Matching / Record Locator Service |
| Provider Registry | Stores information about participating entities. | Patient Demographics Service Patient Matching / Record Locator Service |
| Audit Trail | Captures data necessary to identify who requested data for which patients. | Reporting Services Security and Privacy Services |
| Security I Privacy | Stores information to support the security services, including patient-provider relationships and sealed envelopes. | Security and Privacy Services |
| Terminology | Stores standard terminologies along with the mappings between standard terminologies and local terminologies. | Terminology Management application Transformation and Normalization Service |

Continuing with FIG. 5, the infrastructure layer 340 may use Web Services to provide the primary infrastructure services. Web Services use standard Internet technologies to make functionality available over a network in a standardized programmatic manner. From a technology perspective, Web Services are based on Internet standards, platform agnostic, are widely available, and have complete vendor support. Web Services provide a common mechanism for interoperability among disparate systems, and the key to their utility is their standardization. The value of Web Services is in their ability to reduce the cost of technical integration of systems by applying industry wide Internet standards. This helps reduce delivery time and provides end users the capability to consume service through common user tools. It also provides a common technique for integration both internal and external to the architecture.

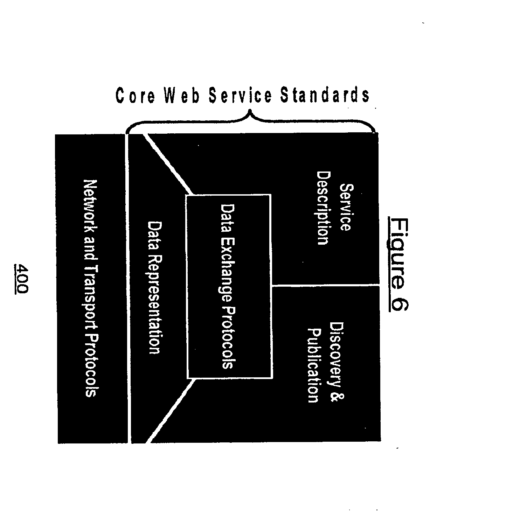

Web services standards can be categorized according to the purpose they serve, and the web services framework 400 for the architecture may be defined as depict in FIG. 6. Web Services standards are continually created, enhanced, and consolidated by numerous organizations and standards bodies. As a result, certain standards are consistently and widely used where they've been adopted into the mainstream, while others may be new or experimental standards used by fewer organizations. For the architecture, utilize web services standards that have been accepted into the mainstream may be used wherever possible.

The Table 4 below defines the standards that may be incorporate into the present invention. The "Category" column refers to the different categories illustrated in the framework 300 of FIG. 5.

| **Table 4** | | | | |
|---|---|---|---|---|
| **Category** | **Standard** | **Name** | **Description** | **Usage in the invention** |
| Service Description | WSDL | Web Services Description Language | Provides a model and an XML format for describing Web Services. Defines Web Services interfaces, data and message types, interaction patterns, and protocol mappings. | WSDL will be used to create the Web Service message structure for messages exchanged between Portals and RHIOs. |
| | WS-Policy | Web Services Policy Framework | Provides a general-purpose model and corresponding syntax to describe and communicate the policies of a Web service. | |
| Discovery & Publication | UDDI | Universal Description, Discovery, and Integration | A set of services supporting the description and discovery of businesses, organizations, and other Web services providers; the Web services they make available; and the technical interfaces which may be used to access those services. | Could be used to publish a RHIO's Web Service information to Providers and other RHIOs. |
| | WSIL | Web Services Inspection Language | Defines a method to discover and locate a list of Web Services published at a particular known receiver's address. | RHIO to RHIO access, RHIO to NHIN access. |
| Data Exchange | SOAP | Simple Object Access Protocol | Defines an XML messaging protocol for basic service interoperability. Provides a simple and lightweight mechanism for exchanging structured and typed information between peers in a decentralized, distributed environment. | Messages exchanged between Portals and RHIO applications will be packaged in a SOAP envelope. |

Continuing with FIG. 5, the Operations Architecture 350 provides for ongoing monitoring and management of the environment. The environment is preferably be managed in a reliable and consistent manner across the central hub and all participating member institutions. Having the capability to support ongoing management and operations is desirable. Since the architecture distributes data to servers at member institutions 210, the operations management capability 350 helps to ensure that each member operates according to consistent, standard service levels. As described above, it is known to manage both the central hub servers as well as (potentially) the servers within member data centers. Taking advantage of hosting and management capability would transfer the complexity of providing a consistent operational service. Regardless of who actually hosts or manages the infrastructure, the Operations Architecture 350 may offer a consistent structure that can use to ensure the appropriate level of operational support for the proposed solution.

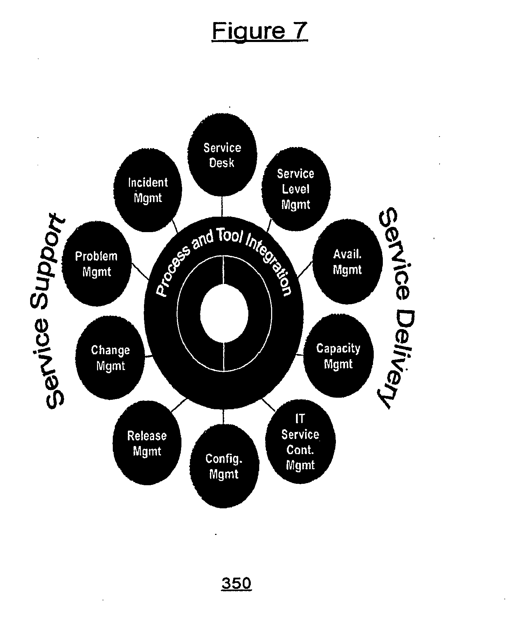

Turning to FIG. 7, the Operations Architecture Framework is illustrated in greater detail and optionally includes various, such as those listed below in Table 5.

| **Table 5** | | |
|---|---|---|
| **Service** | **Definition** | **Relevance for The Architecture** |
| Service Level Management | The process of defining, agreeing, documenting and managing the levels of IT service that are required and cost justified. Service Level Agreements (SLAs) and Operational Level Agreements (OLAs) are typically developed to capture the required levels of service. | The architecture management will use SLAs and OLAs as the basis for evaluating the quality of infrastructure support services. |
| Availability Management | The practice of ensuring that any IT service consistently and cost-effectively delivers the level of availability required by the customer. This practice includes day-to-day operations, administration and maintenance tasks. | The architecture system will require day-to-day maintenance to prevent down-time and ensure service levels. |
| Capacity Management | The process of planning, sizing and controlling infrastructure capacity to satisfy user demand at reasonable cost within target performance levels. This process ensures optimal use of IT resources. | The architecture system will continue to grow, both in the number of participants as well as the different practice settings (e.g., moving from ED to ambulatory). Proactive planning of capacity will be important to ensure that this expected growth does not negatively impact the users. |
| IT Service Continuity Management (Disaster Recovery) | The practice of ensuring that IT services can still provide value even when the primary infrastructure fails. Includes processes and procedures that describe how to recover from an event. Events range from application or system failure to a complete destruction of the premises. In the event of an extreme failure (e.g., natural disaster), services will be restored to support only the minimum business requirements. | Given the events of recent years (e.g., Hurricane Katrina), a disaster recovery plan and procedures is a critical need for the architecture. |
| Configuration Management | The identification, recording and reporting of IT components, including their versions, constituent components and relationships. The scope of configuration management includes hardware, software and associated documentation. Examples of components that would be managed under Configuration Management include hardware, software, network equipment, configurations, processes, procedures, documentation, service level agreements and problem records. | The architecture system has a substantial number of components. Managing these components in an organized and detailed fashion will be an important need. |
| Release Management | The practice of coordinating and managing releases to a live environment. This process balances the need to release changes as quickly as possible to meet business requirements with the need to ensure that changes are implemented in a controlled and systematic way that limits negative impact to the information technology environment. Multiple changes would typically be grouped into a single release to reduce the frequency of changes to the live environment. | The architecture system will continue to grow and change (e.g.. when a new participant is added). Managing the changes to the environment in a way that balances speed with deliberation will be an important need. |
| Change Management | The practice of ensuring that all changes to the infrastructure are carried out in a planned and authorized manner. Key aspects of this practice include confirming the reason for the change, identifying affected services, planning the change, testing the change and having a back out plan in case the change results in an unexpected issue. | Given the intended use of the architecture system, managing changes to the infrastructure is an important need. |
| Problem Management | The process of identifying the root cause of incidents and initiating actions to improve or correct the situation. This process has both reactive and proactive aspects. The reactive aspect is concerned with solving problems in response to incidents, and the proactive aspect is concerned with identifying and solving problems and known errors before an incident occurs. | Problems within the architecture system, particularly those that are persistent, will threaten user acceptance of the system. Problems must be permanently and pro-actively solved. |
| Incident Management | An incident is any event that disrupts the expected standard operation of a system, service or product within the infrastructure. Incident Management aims to restore normal service operation as quickly as possible and minimize the negative impact on business operations. | Incidents within the architecture system will require resolution with minimal impact on business operations. |
| Service Desk | The single point of contact for users of the architecture services. The focal point for reporting incidents and making service requests. The Service Desk also keeps stakeholders informed of service events and actions that are likely to impact their ability to perform day-to-day activities. | The Help Desk - the users will need a place to report incidents and make service requests. |

### Conclusion

While the invention has been described with reference to an exemplary embodiments various additions, deletions, substitutions, or other modifications may be made without departing from the scope of the invention. Accordingly, the invention is not to be considered as limited by the foregoing description, but is only limited by the scope of the appended claims. Further information on the present invention is provided in the attached.

## Claims

1. An interoperable healthcare data exchange platform system (100) for gathering health records (120) of patients from a plurality of disparate locations, the platform system (100) comprising:
a message handling service (130) to request and receive the health records (120) in real time, wherein the message handling service (130) converts the health records (120) to a standard protocol;
a mapping engine (150) that receives the converted health records (120) and maps the converted health records (120) to a standard health terminology;
a data store (160) for receiving and storing the mapped, converted health records (120), wherein the data store (160) stores the mapped, converted health records (120) using the standard protocol and the standard health terminology,
the data store (160) having several core components including a master patient index (170), a provider directory or a record locator service, and the data store (160) containing coded information with a specified identifier for each patient, and
wherein the master patient index (170) includes a strict change control process for patient information to link disparate information of patients received from external systems into one file; and
a information governance application (101) for providing controlled access to the data store (160),
wherein the mapping engine (150) has information about the relevant terminology hierarchy and maintains information about the relationship between terms of the hierarchy, the terminology including vocabularies, code sets, and identifiers to characterize or identify a health provider organization, a location in an organization, a healthcare worker, a medical condition, a health service, a cost of a medical procedure or service, a payer organization or a particular health plan, and
wherein subsumed terms are used by the mapping engine (150) to query terminology mapping (155).

2. Interoperable healthcare data exchange platform system according to claim 1, further comprising a distributed computer system for collection of health records (120) from a health care provider, the computer system comprising
a first database (160) comprising said health records (120) associated with said health care provider;
a records extraction system connected to the first database, the records extraction system comprising:
an extraction module configured to copy selected health records (120), translate the selected health records (120) into a pre-specified format, store the translated health records (120) in a single file, encrypt the file using a pre-specified encryption scheme, and transmit the encrypted file across a network,
a data collection server, wherein the data collection server receives the encrypted file, decrypts the encrypted file using the pre-specified encryption scheme, and translates the file into an internal data storage format, and
an extraction database for temporarily storing the translated health care data records; and
a central storage system connected to the records extraction system for receiving and storing the stored health care data records from the extraction database.

3. Interoperable healthcare data exchange platform system of Claim 2, wherein the records extraction system is adapted to receive an approval signal from the health care provider, and the records extraction system does not forward stored health care data records to the central storage system until the records extraction system receives the approval signal from the health care provider.

4. Interoperable healthcare data exchange platform system of Claim 2, wherein the extraction module is further configured to certify the selected health records (120).

5. Interoperable healthcare data exchange platform system of Claim 4, wherein the certifying of the selected health records (120) by the extraction module comprises verifying that the health records (120) conform with a desired format or wherein the certifying of the selected health records (120) by the extraction module comprises searching the health records (120) for pre-specified data, and rejecting health records (120) containing said pre-specified data.

6. Interoperable healthcare data exchange platform system of Claim 2, wherein the extraction module operates in batch mode to collect the health records (120) periodically or wherein the extraction module operates in real time to collect the health records (120) when updated.

7. Interoperable healthcare data exchange platform system of Claim 2, wherein the healthcare provider is a first healthcare provider, and wherein the distributed computer system further comprises a second database (170) comprising health records (120) associated with a second health care provider, wherein the records extraction system is connected to both the first and the second databases (160, 170), and wherein the central storage system receives and stores the translated health care data records for both the first and the second health care providers.

8. Interoperable healthcare data exchange platform system of Claim 7, wherein the first database stores health records (120) in a first format and the second database (170) stores health records (120) in a second format.

9. Interoperable healthcare data exchange platform system of Claim 2, wherein the extraction module pseudonymizes the health records.

10. Interoperable healthcare data exchange platform system of Claim 2, wherein the central storage system comprises a directory of patients associated with said stored translated health records (120).

11. Interoperable healthcare data exchange platform system of claim 1 further comprising a national health records collection system comprising plurality of electronic health records systems, the national health records collection system comprising
the message handling service (130) implemented on plurality of networked computers connected to said electronic health records systems, wherein each of the electronic health records systems sends associated health records (120) to the message handling service (130), wherein the message handling service (130) comprises a transformation and normalization system attached to said electronic health records systems to take data from plurality of electronic health records systems and normalizes for storage in a central database.

12. Interoperable healthcare data exchange platform system of claim 11, wherein the central database comprises a terminology mapping database (155).

13. Interoperable healthcare data exchange platform system of claim 11, wherein the central database comprises a provider database.

14. Interoperable healthcare data exchange platform system of claim 11, wherein the message handling service (130) comprises plurality of interface adapters for translating individual electronic health records system data into standard format, wherein the standard format preferably is XML, more preferably HL7.

## Patentansprüche

1. Interoperables Gesundheitsdatenaustauschplattformsystem (100) zum Erlangen von Gesundheitsaufzeichnungen bzw. -akten (120) von Patienten von einer Mehrzahl von unterschiedlichen bzw. verstreuten Standorten, wobei das Plattformsystem (100) umfasst:
einen Nachrichtenhandhabungs- bzw. -verarbeitungssystem (130) zum Anfordern und Empfangen der Gesundheitsaufzeichnungen (120) in Echtzeit, wobei das Nachrichtenhandhabungssystem (130) die Gesundheitsaufzeichnungen (120) in ein Standardprotokoll umwandelt;
eine Mapping Engine (150), welche die umgewandelten Gesundheitsaufzeichnungen (120) empfängt und die umgewandelten Gesundheitsaufzeichnungen (120) auf eine Standardgesundheitsterminologie mappt;
einen Datenspeicher (160) zum Empfangen und Speichern der gemappten, umgewandelten Gesundheitsaufzeichnungen (120), wobei der Datenspeicher (160) die gemappten, umgewandelten Gesundheitsaufzeichnungen (120) unter Verwendung des Standardprotokolls und der Standardgesundheitsterminologie speichert,
wobei der Datenspeicher (160) mehrere Kernkomponenten aufweist, die einen Hauptpatientenindex bzw. Master Patient Index (170), ein Anbieterverzeichnis oder einen Aufzeichnungs- bzw. Aktenlokalisiererdienst beinhalten, und wobei der Datenspeicher (160) codierte Informationen mit einem spezifizierten Identifizierer für jeden Patienten enthält, und
wobei der Hauptpatientenindex (170) einen strikten Änderungssteuerungs- bzw. -kontrollprozess für Patienteninformationen enthält, um unterschiedliche bzw. verstreute Informationen von Patienten, die von externen Systemen empfangen werden, in einer Datei zu verlinken bzw. zu verknüpfen; und
eine Information-Governance-Anwendung (101) zum Bereitstellen eines gesteuerten bzw. kontrollierten Zugriffs auf den Datenspeicher (160),
wobei die Mapping Engine (150) Informationen über die relevante Terminologiehierarchie aufweist und Informationen über die Beziehung zwischen Begriffen der Hierarchie aufrechterhält, wobei die Terminologie Vokabeln, Code-Sätze und Identifizierer enthält, um eine Gesundheitsversorgerorganisation, einen Ort in einer Organisation, eine Fachkraft im Gesundheitswesen, einen medizinischen Zustand, einen Gesundheitsdienst, Kosten eines medizinischen Verfahrens oder Dienstes, eine bezahlende Organisation oder einen bestimmten Gesundheitsplan zu charakterisieren oder zu identifizieren, und
wobei zusammengefasste Begriffe von der Mapping Engine (150) verwendet werden, um Terminologie-Mapping (155) abzufragen.

2. Interoperables Gesundheitsdatenaustauschplattformsystem nach Anspruch 1, ferner umfassend ein verteiltes Computersystem zum Sammeln von Gesundheitsaufzeichnungen (120) von einem Gesundheitsleistungserbringer, wobei das Computersystem umfasst
eine erste Datenbank (160), welche die Gesundheitsaufzeichnungen (120) umfasst, die mit dem Gesundheitsleistungserbringer assoziiert bzw. verknüpft sind;
ein Aufzeichnungs- bzw. Aktenextraktionssystem, das mit der ersten Datenbank verbunden ist, wobei das Aufzeichnungsextraktionssystem umfasst:
ein Extraktionsmodul, das konfiguriert ist, die ausgewählten Gesundheitsaufzeichnungen (120) zu kopieren, die ausgewählten Gesundheitsaufzeichnungen (120) in ein vorgegebenes Format zu übersetzen, die übersetzten Gesundheitsaufzeichnungen (120) in einer einzigen Datei zu speichern, die Datei unter Verwendung eines vorgegebenes Verschlüsselungsschemas zu verschlüsseln und die verschlüsselte Datei über ein Netzwerk zu übertragen,
einen Datensammelserver, wobei der Datensammelserver die verschlüsselte Datei empfängt, die verschlüsselte Datei unter Verwendung des vorbestimmten Verschlüsselungsschemas entschlüsselt und die Datei in ein internes Datenspeicherformat übersetzt, und
eine Extraktionsdatenbank zum temporären Speichern der übersetzten Gesundheitsdatenaufzeichnungen; und
ein zentrales Speichersystem, das mit dem Aufzeichnungsextraktionssystem zum Empfangen und Speichern der gespeicherten Gesundheitsdatenaufzeichnungen aus der Extraktionsdatenbank verbunden ist.

3. Interoperables Gesundheitsdatenaustauschplattformsystem nach Anspruch 2, wobei das Aufzeichnungsextraktionssystem angepasst ist, ein Zustimmungssignal von dem Gesundheitsleistungserbringer zu empfangen, und wobei das Aufzeichnungsextraktionssystem keine gespeicherten Gesundheitsdatenaufzeichnungen an das zentrale Speichersystem weiterleitet, bis das Aufzeichnungsextraktionssystem das Zustimmungssignal von dem Gesundheitsleistungserbringer empfängt.

4. Interoperables Gesundheitsdatenaustauschplattformsystem nach Anspruch 2, wobei das Extraktionsmodul ferner konfiguriert ist, die ausgewählten Gesundheitsaufzeichnungen (120) zu bestätigen bzw. zu zertifizieren.

5. Interoperables Gesundheitsdatenaustauschplattformsystem nach Anspruch 4, wobei das Bestätigen bzw. Zertifizieren der ausgewählten Gesundheitsaufzeichnungen (120) durch das Extraktionsmodul das Verifizieren umfasst, dass die Gesundheitsaufzeichnungen (120) mit einem gewünschten Format übereinstimmen, oder wobei das Bestätigen bzw. Zertifizieren der ausgewählten Gesundheitsaufzeichnurigen (120) durch das Extraktionsmodul das Durchsuchen der Gesundheitsaufzeichnungen (120) nach vorbestimmten Daten und Abweisen der Gesundheitsaufzeichnungen (120), die diese vorbestimmten Daten enthalten, umfasst.

6. Interoperables Gesundheitsdatenaustauschplattformsystem nach Anspruch 2, wobei das Extraktionsmodul im Batch-Modus arbeitet, um die Gesundheitsaufzeichnungen (120) periodisch zu sammeln, oder wobei das Extraktionsmodul in Echtzeit arbeitet, um die Gesundheitsaufzeichnungen (120) zu sammeln, wenn sie aktualisiert sind bzw. werden.

7. Interoperables Gesundheitsdatenaustauschplattformsystem nach Anspruch 2, wobei der Gesundheitsleistungserbringer ein erster Gesundheitsleistungserbringer ist und wobei das verteilte Computersystem ferner eine zweite Datenbank (170) umfasst, die Gesundheitsaufzeichnungen (120) umfasst, die mit einem zweiten Gesundheitsleistungserbringer assoziiert bzw. verknüpft sind, wobei das Aufzeichnungsextraktionssystem mit sowohl der ersten als auch der zweiten Datenbank (160, 170) verbunden ist und wobei das zentrale Speichersystem die übersetzten Gesundheitsdatenaufzeichnungen für sowohl den ersten als auch den zweiten Gesundheitsleistungserbringer empfängt und speichert.

8. Interoperables Gesundheitsdatenaustauschplattformsystem nach Anspruch 7, wobei die erste Datenbank Gesundheitsaufzeichnungen (120) in einem ersten Format speichert und die zweite Datenbank (170) Gesundheitsaufzeichnungen (120) in einem zweiten Format speichert.

9. Interoperables Gesundheitsdatenaustauschplattformsystem nach Anspruch 2, wobei das Extraktionsmodul die Gesundheitsaufzeichnungen pseudonymisiert.

10. Interoperables Gesundheitsdatenaustauschplattformsystem nach Anspruch 2, wobei das zentrale Speichersystem ein Patientenverzeichnis umfasst, das mit den gespeicherten übersetzten Gesundheitsaufzeichnungen (120) assoziiert bzw. verknüpft ist.

11. Interoperables Gesundheitsdatenaustauschplattformsystem nach Anspruch 1, ferner umfassend ein nationales bzw. staatliches Gesundheitsaufzeichnungensammelsystem, das eine Mehrzahl von elektronischen Gesundheitsaufzeichnungssystemen umfasst, wobei das nationale Gesundheitsaufzeichnungensammelsystem umfasst
das Nachrichtenhandhabungssystem (130), das auf einer Mehrzahl von vernetzten Computer implementiert ist, die mit den elektronischen Gesundheitsaufzeichnungssystemen verbunden sind, wobei jedes der elektronischen Gesundheitsaufzeichnungssysteme assoziierte bzw. verknüpfte Gesundheitsaufzeichnungen (120) an das Nachrichtenhandhabungssystem (130) sendet, wobei das Nachrichtenhandhabungssystem (130) ein Umwandlungs- und Normalisierungssystem umfasst, das an die elektronischen Gesundheitsaufzeichnungssystemen angefügt ist, um Daten aus Mehrzahl von elektronischen Gesundheitsaufzeichnungssystemen zu nehmen und zur Speicherung in einer zentralen Datenbank zu normalisieren.

12. Interoperables Gesundheitsdatenaustauschplattformsystem nach Anspruch 11, wobei die zentrale Datenbank eine Terminologie-Mapping-Datenbank (155) umfasst.

13. Interoperables Gesundheitsdatenaustauschplattformsystem nach Anspruch 11, wobei die zentrale Datenbank eine Anbieter- bzw. Erbringerdatenbank umfasst.

14. Interoperables Gesundheitsdatenaustauschplattformsystem nach Anspruch 11, wobei das Nachrichtenhandhabungssystem (130) eine Mehrzahl von Schnittstellenadapter zum Übersetzen individueller bzw. einzelner elektronischer Gesundheitsaufzeichnungssystemdaten in Standardformat umfasst, wobei das Standardformat vorzugsweise XML ist, noch bevorzugter HL7.

## Revendications

1. Système de plate-forme d'échange de données de soins de santé interopérable (100) pour rassembler des dossiers médicaux (120) de patients depuis une pluralité d'emplacements disparates, le système de plate-forme (100) comprenant :
un service de gestion de messages (130) pour demander et recevoir les dossiers médicaux (120) en temps réel, dans lequel le service de gestion de messages (130) convertit les dossiers médicaux (120) en un protocole standard ;
un moteur de mise en correspondance (150) qui reçoit les dossiers médicaux convertis (120) et met en correspondance les dossiers médicaux convertis (120) avec une terminologie de santé standard ;
un magasin de données (160) pour recevoir et stocker les dossiers médicaux convertis mis en correspondance (120), dans lequel le magasin de données (160) stocke les dossiers médicaux convertis mis en correspondance (120) en utilisant le protocole standard et la terminologie de santé standard,
le magasin de données (160) ayant plusieurs composants centraux incluant un index patients maître (170), un annuaire de fournisseurs ou un service localisateur de dossiers, et le magasin de données (160) contenant des informations codées avec un identifiant spécifié pour chaque patient, et
dans lequel l'index patients maître (170) inclut un processus de contrôle de changement strict pour les informations concernant les patients pour lier des informations disparates concernant des patients reçues de systèmes externes dans un fichier ; et
une application de gouvernance d'informations (101) pour fournir un accès contrôlé au magasin de données (160),
dans lequel le moteur de mise en correspondance (150) a des informations concernant la hiérarchie de terminologie pertinente et maintient des informations concernant le rapport entre des termes de la hiérarchie, la terminologie incluant des vocabulaires, des ensembles de code et des identifiants pour caractériser ou identifier une organisation de fournisseur de santé, un emplacement dans une organisation, un travailleur de soins de santé, une affection médicale, un service de santé, un coût d'une procédure ou d'un service médical(e), une organisation de payeur ou un plan de santé particulier, et
dans lequel des termes inclus dans une classification sont utilisés par le moteur de mise en correspondance (150) pour interroger une mise en correspondance de terminologie (155).

2. Système de plate-forme d'échange de données de soins de santé interopérable selon la revendication 1, comprenant en outre un système informatique distribué pour le recueil de dossiers médicaux (120) auprès d'un fournisseur de soins de santé, le système informatique comprenant
une première base de données (160) comprenant lesdits dossiers médicaux (120) associés audit fournisseur de soins de santé ;
un système d'extraction de dossiers connecté à la première base de données, le système d'extraction de dossiers comprenant :
un module d'extraction configuré pour copier des dossiers médicaux sélectionnés (120), traduire les dossiers médicaux sélectionnés (120) en un format spécifié au préalable, stocker les dossiers médicaux traduits (120) dans un seul fichier, encrypter le fichier en utilisant un programme d'encryptage spécifié au préalable et transmettre le fichier encrypté sur un réseau,
un serveur de recueil de données, dans lequel le serveur de recueil de données reçoit le fichier encrypté, décrypte le fichier encrypté en utilisant le programme d'encryptage spécifié au préalable et traduit le fichier en un format de stockage de données interne, et
une base de données d'extraction pour stocker temporairement les dossiers de données de soins de santé traduits ; et
un système de stockage central connecté au système d'extraction de dossiers pour recevoir et stocker les dossiers de données de soins de santé stockés de la base de données d'extraction.

3. Système de plate-forme d'échange de données de soins de santé interopérable selon la revendication 2, dans lequel le système d'extraction de dossiers est adapté à recevoir un signal d'approbation du fournisseur de soins de santé et le système d'extraction de dossiers ne transfère pas de dossiers de données de soins de santé stockés au système de stockage central jusqu'à ce que le système d'extraction de dossiers reçoive le signal d'approbation du fournisseur de soins de santé.

4. Système de plate-forme d'échange de données de soins de santé interopérable selon la revendication 2, dans lequel le module d'extraction est en outre configuré pour certifier les dossiers médicaux sélectionnés (120).

5. Système de plate-forme d'échange de données de soins de santé interopérable selon la revendication 4, dans lequel la certification des dossiers médicaux sélectionnés (120) par le module d'extraction comprend vérifier que les dossiers médicaux (120) se conforment à un format souhaité ou dans lequel la certification des dossiers médicaux sélectionnés (120) par le module d'extraction comprend parcourir les dossiers médicaux (120) à la recherche de données spécifiées au préalable et rejeter les dossiers médicaux (120) contenant lesdites données spécifiées au préalable.

6. Système de plate-forme d'échange de données de soins de santé interopérable selon la revendication 2, dans lequel le module d'extraction opère en mode par lot pour recueillir les dossiers médicaux (120) périodiquement ou dans lequel le module d'extraction opère en temps réel pour recueillir les dossiers médicaux (120) lors de la mise à jour.

7. Système de plate-forme d'échange de données de soins de santé interopérable selon la revendication 2, dans lequel le fournisseur de soins de santé est un premier fournisseur de soins de santé et dans lequel le système informatique distribué comprend en outre une seconde base de données (170) comprenant des dossiers médicaux (120) associés à un second fournisseur de soins de santé, dans lequel le système d'extraction de dossiers est connecté à la fois à la première et la seconde bases de données (160, 170), et dans lequel le système de stockage central reçoit et stocke les dossiers de données de soins de santé traduits pour à la fois le premier et le second fournisseurs de soins de santé.

8. Système de plate-forme d'échange de données de soins de santé interopérable selon la revendication 7, dans lequel la première base de données stocke les dossiers médicaux (120) dans un premier format et la seconde base de données (170) stocke les dossiers médicaux (120) dans un second format.

9. Système de plate-forme d'échange de données de soins de santé interopérable selon la revendication 2, dans lequel le module d'extraction pseudonymise les dossiers médicaux.

10. Système de plate-forme d'échange de données de soins de santé interopérable selon la revendication 2, dans lequel le système de stockage central comprend un annuaire de patients associés auxdits dossiers médicaux traduits stockés (120).

11. Système de plate-forme d'échange de données de soins de santé interopérable selon la revendication 1, comprenant en outre un système de recueil de dossiers médicaux national comprenant une pluralité de systèmes de dossiers médicaux électroniques, le système de recueil de dossiers médicaux national comprenant le service de gestion de messages (130) mis en oeuvre sur une pluralité d'ordinateurs mis en réseau connectés auxdits systèmes de dossiers médicaux électroniques, dans lequel chacun des systèmes de dossiers médicaux électroniques envoie les dossiers médicaux associés (120) au service de gestion de messages (130), dans lequel le service de gestion de messages (130) comprend un système de transformation et normalisation relié auxdits systèmes de dossiers médicaux électroniques pour prélever des données de la pluralité de systèmes de dossiers médicaux électroniques et les normaliser pour le stockage dans une base de données centrale.

12. Système de plate-forme d'échange de données de soins de santé interopérable selon la revendication 11, dans lequel la base de données centrale comprend une base de données de mise en correspondance de terminologie (155).

13. Système de plate-forme d'échange de données de soins de santé interopérable selon la revendication 11, dans lequel la base de données centrale comprend une base de données de fournisseurs.

14. Système de plate-forme d'échange de données de soins de santé interopérable selon la revendication 11, dans lequel le service de gestion de messages (130) comprend une pluralité d'adaptateurs d'interface pour traduire des données de système de dossiers médicaux électronique individuel au format standard, dans lequel le format standard est de préférence XML, plus préférablement HL7.
